(19) Europäisches Patentamt
European Patent Office
Office européen des brevets

(11) Publication number: **0 067 124**
**B1**

(12) # EUROPEAN PATENT SPECIFICATION

(45) Date of publication of patent specification: **02.05.85**

(21) Application number: **82810239.2**

(22) Date of filing: **01.06.82**

(51) Int. Cl.$^4$: **C 07 C 59/88,** C 07 C 51/60, C 07 C 69/738, C 07 C 153/11, C 07 C 103/58, C 07 D 295/18, C 07 C 51/00, C 07 C 67/00, C 07 C 102/02

(54) **Production of 5,5-disubstituted 3-oxo-pent-4-enoic acids, their halides, esters, thioesters and amides; and their use in antifungal and antibacterial compositions.**

(30) Priority: **05.06.81 GB 8117357**

(43) Date of publication of application:
**15.12.82 Bulletin 82/50**

(45) Publication of the grant of the patent:
**02.05.85 Bulletin 85/18**

(84) Designated Contracting States:
**AT BE CH DE FR GB IT LI NL SE**

(56) References cited:
**US-A-4 291 057**

**Chemical Abstracts vol. 64, no. 9 25 April 1966 Columbus, Ohio, USA F. POCHAT et al. "Polyhalodivinyl ketones. Synthesis and investigation of two tetra- and pentachlorinated pentadienones" columns 12540e to 12541a**

(73) Proprietor: **CIBA-GEIGY AG**
**Postfach**
**CH-4002 Basel (CH)**

(72) Inventor: **Bentley, Robert Leonard, Dr.**
**34 Eddisbury Avenue**
**Urmston Manchester M31 2QJ (GB)**
Inventor: **Dingwall, John Grey, Dr.**
**Niederholzstrasse 51**
**CH-4125 Riehen (CH)**
Inventor: **Gainer, James, Dr.**
**8 Moorgate Drive**
**Astley, Tyldesley, Manchester M29 7DG (GB)**
Inventor: **Tuck, Brian, Dr.**
**91, Seal Road Bramhall**
**Stockport Cheshire SK7 2LE (GB)**

(56) References cited:
**Chemical Abstracts vol. 78, no. 3 22 January 1973 Columbus, Ohio, USA F. POCHAT et al. "Polyhalo-alpha,alpha'-unsaturated ketones and their derivatives. II. Preparation and properties of polychloro-gamma-unsaturated beta-keto esters" page 337, column 2 to page 338, column 1, abstract no. 15449n**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 067 124 B1

Courier Press, Leamington Spa, England.

**Description**

The present invention relates to a process for the production of 5,5-disubstituted 3-oxopent-4-enoic acids and their acid halidies, esters, thioesters and amides; to the new products of this process; and to their use in antifungal and antibacterial compositions.

The present invention provides a process for the production of a compound having the formula:

$$R^2R^3C = CHCOCH_2COZ \qquad \text{I}$$

wherein $R^2$ is fluorine, chlorine, or bromine; $R^3$ is hydrogen, fluorine, chlorine or bromine, or a group of formula —$CR^4R^5R^6$ wherein $R^4$,$R^5$ and $R^6$ are fluorine, chlorine or bromine; and Z is —AY or $X^1$, wherein A is —O—, —S— or —$NR^7$ wherein $R^7$ is hydrogen, $C_{1-10}$-straight — or branch chain alkyl or $C_{3-4}$-straight or branch chain alkenyl, Y is hydrogen, $C_{1-18}$-straight or branch chain alkyl optionally interrupted by one to five oxygen atoms and optionally substituted by one to five chlorine or bromine atoms, or by a group having the formula —$OR^8$ or the formula —$CO_2R^9$ wherein $R^8$ is hydrogen, $C_{3-12}$-cycloalkyl, straight- or branch chain $C_{3-18}$-alkenyl, $C_{6-10}$-aryl or $C_{7-13}$-aralkyl and $R^9$ is hydrogen, $C_{1-18}$-straight- or branch chain alkyl, or straight- or branch chain $C_{3-18}$-alkenyl, or Y is $C_{3-18}$straight or branch chain alkenyl, $C_{3-18}$ straight- or branch chain alkynyl, $C_{3-12}$-cyclo alkyl, $C_{7-13}$-aralkyl, or $C_{6-10}$-aryl which is optionally substituted by one to three $C_{1-9}$ straight- or branch chain alkyl, halogen, nitro, $CF_3$, or $SO_3H$, or groups —$OR^{10}$ wherein $R^{10}$ is hydrogen or $C_{1-4}$ straight- or branch chain alkyl, provided that the total number of carbon atoms in the group —AY is from 0 to 20; or $R^7$ and Y together may form a $C_{2-7}$ polymethylene chain optionally substituted by one or two $C_{1-4}$ alkyl groups and optionally interrupted by —O—, —S— or —$NR^{11}$ groups wherein $R^{11}$ is $C_{1-4}$ straight or branch chain alkyl; and $X^1$ is Cl or Br; comprising (a) reacting, optionally in the presence of a solvent and an inorganic or organic acid-binding agent, a compound having the formula II:

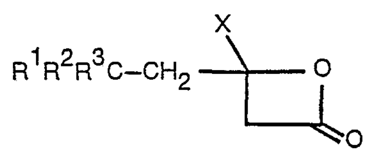

II

wherein $R^2$ and $R^3$ have their previous significance; $R^1$ is fluorine, chlorine or bromine; and X is chlorine, bromine, or iodine with the proviso that, when X is chlorine, none of $R^1$, $R^2$, $R^3$, $R^4$, $R^5$ and $R^6$ can be bromine; with a compound of formula H—Z (III) wherein Z has its previous significance; and (b) when Z is —AY and —AY is —OH, the product of step a) is optionally reacted with a compound (IV) capable of converting a carboxyl group into the corresponding carboxylic acid chloride or -bromide, thereby producing a compound of formula I wherein Z is- $X^1$.

It will be appreciated by those skilled in the art that when X is chlorine, none of $R^1$, $R^2$, $R^3$, $R^4$, $R^5$ and $R^6$ can be a bromine atom as such compounds of formula II cannot be produced by known methods.

When $R^7$ is a $C_1$—$C_{10}$ straight- or branch chain alkyl group, it may be, for example, a methyl, ethyl, n-propyl, isopropyl, n-butyl, sec.-butyl, t-butyl, n-pentyl, n-hexyl, n-heptyl, n-octyl, t-octyl or n-decyl residue, but is preferably a $C_1$—$C_6$ alkyl residue.

When $R^7$ is a $C_3$—$C_4$ straight- or branch chain alkenyl group, it may be a propenyl or butenyl residue.

When Y and/or $R^9$ is a $C_1$—$C_{18}$ straight- or branch chain alkyl residue it may be, for example, a methyl, ethyl, n-propyl, i-propyl, n-butyl, s-butyl, t-butyl, n-pentyl, n-hexyl, n-heptyl, n-octyl, t-octyl, 2-ethylhexyl, n-decyl, n-dodecyl, n-tetradecyl, n-hexadecyl or n-octadecyl residue.

When Y or $R^8$ or $R^9$ is a $C_3$—$C_{18}$ straight- or branched chain alkenyl residue it may be, for example, a prop-2-en-1-yl, butenyl, pentenyl, hexenyl, hexadienyl, oct-1-en-3-yl, dodecenyl or octadecenyl residue.

When Y is a $C_3$—$C_{18}$ straight- or branch chain alkynyl residue it may be, for example, a propargyl, but-3-yn-1-yl, pentynyl, hexynyl or octadecynyl residue.

When Y or $R^8$ is a $C_3$—$C_{12}$ cycloalkyl residue it may be, for example, a cyclopropyl, cyclobutyl, cyclopentyl, cyclohexyl, cycloheptyl, cyclooctyl or a cyclododecyl residue.

When Y or $R^8$ is a $C_7$—$C_{13}$ aralkyl residue it may be, for example, a benzyl, phenethyl α,α-dimethylbenzyl, or benzhydryl residue.

When Y or $R^8$ is a $C_6$—$C_{10}$ aryl residue it may be, for example, a phenyl or naphthyl residue.

When Y is a substituted $C_1$—$C_{18}$ alkyl residue, it may be e.g. a chloromethyl, chloroethyl, bromodecyl or chlorooctadecyl, carboxymethyl, carboxyethyl, carboxyoctadecyl, methoxycarbonylmethyl, ethoxycarbonylethyl, 2-cyclohexyloxyethyl, 2-phenoxyethyl or 2-benzyloxyethyl residue.

When Y is a $C_1$—$C_{18}$ alkyl residue interrupted by one or more oxygen atoms, it may be e.g. a residue having the formula —$CH_2CH_2OCH_3$, —$CH_2CH_2O(CH_2)_3CH_3$, —$CH_2CH_2O(CH_2)_7CH_3$, —$CH_2CH_2OCH_2CH_2OCH_2CH_3$ or —$CH_2CH_2OCH_2CH_2OCH_2CH(C_2H_5)(CH_2)_3CH_3$.

When Y is a substituted $C_6$—$C_{10}$ aryl residue, it may be e.g. a tolyl, xylyl, p-nonylphenyl, p-chlorophenyl, m-trifluoromethylphenyl or p-methoxyphenyl residue.

$C_1$—$C_4$ alkyl groups $R^{10}$ and $R^{11}$ are e.g. methyl, ethyl, n-propyl, isopropyl, sec-butyl or tert-butyl groups.

When $R^7$ and Y together form a $C_2$—$C_7$ polymethylene chain optionally interrupted by O, S or $NR^{11}$, this chain may be, for example, $-(CH_2)_2-$, $-(CH_2)_3-$, $-(CH_2)_4-$, $-(CH_2)_5-$, $-(CH_2)_6-$, $-(CH_2)_7-$, $-(CH_2)_2O(CH_2)_2-$, $-(CH_2)_2S(CH_2)_2-$, $-(CH_2)_2NR^{11}(CH_2)_2-$ or $-CH_2CH(CH_3)OCH(CH_3)CH_2-$ group.

Step a) may be carried out with or without a solvent. The solvent may be inert under the conditions of the reaction or may be an excess of one of the reagents. Examples of suitable solvents are hydrocarbons, carbon tetrachloride, methylene chloride, diethylether, acetone, dioxan, methanol, ethanol, water, acetic acid, aqueous dioxan, aqueous acetone and sulphuric acid.

Step a) may optionally be carried out in the presence of an inorganic or organic acid binding agent. Examples of suitable acid binding agents are sodium acetate, sodium hydrogen carbonate, sodium methoxide, triethylamine, ethyl-di-isopropylamine and pyridine. Alternatively an excess of the reagent HZ may be used as the acid binding agent provided that A is $-NR^7$ where $R^7$ is defined above.

Step a) may also be carried out by preparing the anion of HZ with a base particularly where A is —O— or —S—. The reaction is then preferably carried out using 3 or more equivalents of this salt to one of the lactone.

The temperature in step a) may be from −20°C. to 150°C. preferably 0°C. to 100°C.

In a first preferred process according to the invention, when A is —O—, the lactone of formula II is reacted with an excess of alcohol (III) as solvent, and in the presence of an excess of acid binding agent e.g. sodium hydrogen carbonate. The temperature is conveniently from 0 to 120°C, preferably from 20 to 80°C.

In a second, equally preferred process, when A is $-NR^7$:

i) equimolar quantities of the lactone (II) and the amine (III) are stirred together in an inert solvent, e.g. methylene chloride or diethyl ether, in the presence of an excess of acid binding agent e.g. sodium hydrogen carbonate.

The reaction temperature applied is preferably one between 0°C. and the boiling point of the solvent;

ii) the lactone (II) is reacted with the amine (III) in aqueous organic solvent e.g. aqueous tetrahydrofuran or aqueous dioxan. An excess of the amine is used as the acid binding agent. The preferred reaction temperature is from 0° to 35°C; or

iii) the lactone (II) and the amine (III) are reacted together in an organic acid solvent, e.g. acetic acid, in the presence of at least three equivalents of the corresponding salt e.g. sodium acetate. Preferably, the reaction temperature is within the range of from 20 to 30°C, especially 25°C.

In the optional step (b) of the process of the invention, the product of step a) when AY is OH is reacted, optionally in the presence of an inert solvent, conveniently at a temperature of from 0 to 100°C. (preferably from 0 to 40°C) with the compound (IV). Examples of suitable compounds (IV) are $PCl_5$, $PBr_5$, $POCl_3$ and $SOCl_2$. Inert solvents suitable for use in step b) are hydrocarbons, halocarbons e.g. $CCl_4$ and methylene chloride.

In the above processes, it is particularly preferred to use lactones of formula II wherein $R_1$, $R_2$, $R_3$ and X are each chlorine or bromine, especially chlorine.

The starting materials of formula II may be produced by reacting diketene with a compound $R^1R^2R^3CX$ in the presence of an agent capable of forming free radicals. The starting materials of formula III are compounds which are well known per se.

Examples of compound of formula I are:

Methyl 5,5-dichloro-3-oxopent-4-enoate
Isopropyl 5,5-dichloro-3-oxopent-4-enoate
n-Butyl 5,5-dichloro-3-oxopent-4-enoate
tert-Butyl 5,5-dichloro-3-oxopent-4-enoate
n-Octyl 5,5-dichloro-3-oxopent-4-enoate
2-Ethylhexyl 5,5-dichloro-3-oxopent-4-enoate
n-Octadecyl 5,5-dichloro-3-oxopent-4-enoate
2-n-Butoxyethyl 5,5-dichloro-3-oxopent-4-enoate
2-Chloroethyl 5,5-dichloro-3-oxopent-4-enoate
3-Chloropropyl 5,5-dichloro-3-oxopent-4-enoate
2,2,2-Trichloroethyl 5,5-dichloro-3-oxopent-4-enoate
Allyl 5,5-dichloro-3-oxopent-4-enoate
Propargyl 5,5-dichloro-3-oxopent-4-enoate
Cyclohexyl 5,5-dichloro-3-oxopent-4-enoate
Benzyl 5,5-dichloro-3-oxopent-4-enoate
Phenyl 5,5-dichloro-3-oxopent-4-enoate
o-Tolyl 5,5-dichloro-3-oxopent-4-enoate
p-tert-Butylphenyl 5,5-dichloro-3-oxopent-4-enoate
2-Fluorophenyl 5,5-dichloro-3-oxopent-4-enoate
3-Chlorophenyl 5,5-dichloro-3-oxopent-4-enoate
4-Bromophenyl 5,5-dichloro-3-oxopent-4-enoate
2,4-Dimethylphenyl 5,5-dichloro-3-oxopent-4-enoate
3,4-Dichlorophenyl 5,5-dichloro-3-oxopent-4-enoate

4-Methoxyphenyl 5,5-dichloro-3-oxopent-4-enoate
3-Nitrophenyl 5,5-dichloro-3-oxopent-4-enoate
4-Chloro-2-methylphenyl 5,5-dichloro-3-oxopent-4-enoate
2,4,6-Trimethylphenyl 5,5-dichloro-3-oxopent-4-enoate
1-Naphthyl 5,5-dichloro-3-oxopent-4-enoate
5,5-Dibromo-3-oxopent-4-enoic acid
Methyl 5,5-dibromo-3-oxopent-4-enoate
Phenyl 5,5-dibromo-3-oxopent-4-enoate
Methyl 5-chloro-6,6,6-trifluoro-3-oxohex-4-enoate
5,5-Dichloro-3-oxopent-4-enamide
N-Methyl-5,5-dichloro-3-oxopent-4-enamide
N-Ethyl-5,5-dichloro-3-oxopent-4-enamide
N-n-Propyl-5,5-dichloro-3-oxopent-4-enamide
N-Isopropyl-5,5-dichloro-3-oxopent-4-enamide
N-n-Butyl-5,5-dichloro-3-oxopent-4-enamide
N-tert-Butyl-5,5-dichloro-3-oxopent-4-enamide
N-n-Octyl-5,5-dichloro-3-oxopent-4-enamide
N-n-Hexadecyl-5,5-dichloro-3-oxopent-4-enamide
N-n-Octadecyl-5,5-dichloro-3-oxopent-4-enamide
N-Allyl-5,5-dichloro-3-oxopent-4-enamide
N-Propargyl-5,5-dichloro-3-oxopent-4-enamide
N-Cyclopropyl-5,5-dichloro-3-oxopent-4-enamide
N-Cyclohexyl-5,5-dichloro-3-oxopent-4-enamide
N-Benzyl-5,5-dichloro-3-oxopent-4-enamide
N-Benzhydryl-5,5-dichloro-3-oxopent-4-enamide
N,N-Dimethyl-5,5-dichloro-3-oxopent-4-enamide
N,N-Diethyl-5,5-dichloro-3-oxopent-4-enamide
N,N-Diallyl-5,5-dichloro-3-oxopent-4-enamide
N-Methyl-N-phenyl-5,5-dichloro-3-oxopent-4-enamide
N-(2-Hydroxyethyl)-5,5-dichloro-3-oxopent-4-enamide
N-(7-Hydroxyhept-1-yl)-5,5-dichloro-3-oxopent-4-enamide
N-(Carboxymethyl)-5,5-dichloro-3-oxopent-4-enamide
N-(3-n-butoxyprop-1-yloxycarbonyl-methyl)-5,5-dichloro-3-oxopent-4-enamide
N-(Ethoxycarbonylmethyl)-5,5-dichloro-3-oxopent-4-enamide
N-Dodecyloxycarbonylmethyl)-5,5-dichloro-3-oxopent-4-enamide
N-(1-Carboxyethyl)-5,5-dichloro-3-oxopent-4-enamide
N-(2-Carboxyethyl)-5,5-dichloro-3-oxopent-4-enamide
N-(3-Carboxyprop-1-yl)-5,5-dichloro-3-oxopent-4-enamide
N-(1-Carboxy-2-methylprop-1-yl)-5,5-dichloro-3-oxopent-4-enamide
N-(5-Ethoxycarbonylpent-1-yl)-5,5-dichloro-3-oxopent-4-enamide
N-Phenyl-5,5-dichloro-3-oxopent-4-enamide
N-p-Tolyl-5,5-dichloro-3-oxopent-4-enamide
N-(4-Fluorophenyl)-5,5-dichloro-3-oxopent-4-enamide
N-(3-Chlorophenyl)-5,5-dichloro-3-oxopent-4-enamide
N-(2-Bromophenyl)-5,5-dichloro-3-oxopent-4-enamide
N-(4-Chloro-2-methylphenyl)-5,5-dichloro-3-oxopent-4-enamide
N-(2,4-Dimethylphenyl)-5,5-dichloro-3-oxopent-4-enamide
N-(2,6-Dimethylphenyl)-5,5-dichloro-3-oxopent-4-enamide
N-(3-Methoxyphenyl)-5,5-dichloro-3-oxopent-4-enamide
N-(4-Methoxyphenyl)-5,5-dichloro-3-oxopent-4-enamide
N-(5,5-Dichloro-3-oxopent-4-enoyl)-morpholine
N-(5,5-Dichloro-3-oxopent-4-enoyl)-3,5-dimethylmorpholine
N-(5,5-Dichloro-3-oxopent-4-enoyl)-pyrrolidine
N-(5,5-Dichloro-3-oxopent-4-enoyl)-piperidine
N-Methyl-5,5-dibromo-3-oxopent-4-enamide
N-Phenyl-5,5-dibromo-3-oxopent-4-enamide
N,N-Dimethyl-5,5-dibromo-3-oxopent-4-enamide
N-Methyl-5,6,6,6-tetrachloro-3-oxohex-4-enamide
Methyl 5,5-dichloro-3-oxopent-4-enethiolate
Phenyl 5,5-dichloro-3-oxopent-4-enthiolate
Ethoxycarbonylmethyl 5,5-dichloro-3-oxopent-4-enethiolate
5,5-Dichloro-3-oxopent-4-enoyl chloride
5,5-Dichloro-3-oxopent-4-enoyl bromide
5,5-Dibromo-3-oxopent-4-enoyl chloride
5,5-Dibromo-3-oxopent-4-enoyl bromide

as well as the known compounds:

5,5-dichloro-3-oxopent-4-enoic acid

Ethyl 5,5-dichloro-3-oxopent-4-enoate, which are described by Pochat et al. C.R. Acad. Sci. Paris Ser. C. 1966, *261*, 135 and Pochat et al. Bull. Soc. Chim. France 1972, 3151.

With two exceptions, the compounds of formula I produced according to the process of the present invention are novel, therefore the present invention also provides new compounds having the formula:

$$R^2R^3C = CHCOCH_2CO—Z \qquad IA$$

wherein Z, $R^2$ and $R^3$ have their previous significance except that, when $R^2$ and $R^3$ are each chlorine, and Z is —AY wherein A is —O— then Y is not hydrogen or ethyl.

Preferred compounds of formula IA are those wherein Z is —AY in which A is —O— or —$NR^7$ and $R^2$, $R^3$ and Y have their previous significance. More preferred are those compounds wherein Z is —AY in which A is —O— or —$NR^7$, $R^2$ and $R^3$ are chlorine and Y has its previous significance. Most preferred are compounds of formula I wherein Z is —AY in which A is —O—, $R^2$ and $R^3$ are each chlorine and Y is H or $C_1$—$C_6$ alkyl optionally substituted by from 1 to 5 chlorine atoms; and those wherein A is —$NR^7$ wherein $R^7$ is H or $C_1$—$C_6$ alkyl, $R^2$ and $R^3$ are each chlorine and Y has its previous significance.

The compounds of formula I have been found to be active as antibacterial and antifungal agents. Moreover, these compounds are useful as intermediates; for example for biologically active compounds such as the pyrazolyl thiophosphates described in German Patent Specification 2855256.

Accordingly, the present invention also provides an antibacterial and antifungal composition comprising, as active ingredient, a compound of formula I.

The compounds of the formula I possess for practical purposes a very advantageous microbicidal spectrum for protecting cultivated plants. Examples of cultivated plants within the scope of this invention are: cereals, maize, rice, vegetables, sugar-beet, soya, ground nuts, fruit trees, ornamentals, vines, hops, cucumber plants (cucumber, marrows, melons), solanaceae, such as potatoes, tobacco plants and tomatoes, and also banana, cocoa and natural rubber plants.

With the active compounds of the formula I it is possible to inhibit or destroy the fungi which occur in plants or parts of plants (fruit, blossoms, leaves, stems, tubers, roots) in these and also related crops of useful plants, and also to protect from attack by such fungi the parts of plants which grow later. The active compounds are effective against the phytopathogenic fungi which belong to the following classes:

Ascomycetes (e.g. Helminthosporium); Basidiomycetes, in particular rust fungi (e.g. Puccinia); fungi imperfecti (e.g. Moniliales e.g. Cercospora); and against the Oomycetes belonging to the class of the Phycomycetes, such as Phytophthora. The compounds of the formula I are also effective against phytopathogenic bacteria such as Xanthomonas sp., Pseudomonas sp., Erwinia and Corynebacterium. In addition, the compounds of the formula I possess a systemic action. They can also be used as seed dressing agents for protecting seeds (fruit, tubers, grains) and plant cuttings from infection and from phytopathogenic microorganisms which occur in the soil.

The present invention still further provides an antimicrobial composition comprising an amount of a compound of formula I which is effective against the growth of bacteria and fungi which are pathogenic to plants.

The antimicrobial compositions according to the invention are prepared in a manner which is in itself known by intimate mixing and grinding of active compounds of the formula I with suitable carriers, if desired with addition of dispersing agents or solvents which are inert towards the active compounds. The active compounds may exist, and be used, in the following processing forms:

Solid processing forms: dusting agents, sprinkling agents, granules, coated granules, impregnated granules and homogeneous granules.

Active compound concentrates which are dispersible in water: wettable powders, pastes and emulsions.

Liquid processing forms: solutions.

In order to prepare solid processing forms (dusting agents, sprinkling agents and granules), the active compounds are mixed with solid carriers. Examples of carriers which can be used are kaolin, talc, bolus, loess, chalk, limestone, lime grits, attapulgite, dolomite, diatomaceous earth, precipitated silica, alkaline earth metal silicates, sodium and potassium aluminosilicates (feldspars and micas), calcium and magnesium sulphates, magnesium oxide, ground plastics, fertilisers, such as ammonium sulphate, ammonium phosphate, ammonium nitrate and urea, ground vegetable products, such as cereal flour, bark flour, wood flour, nutshell flour, cellulose powder, plant extract residues, active charcoal and the like, in each case on their own or as mixtures with one another.

Granules can be prepared by, for example, dissolving the active compounds in an organic solvent, applying the solution thus obtained to a granulated material, for example attapulgite, silica, granicalcium or bentonite and then again evaporating the organic solvent.

It is also possible to prepare polymer granules by, for example, impregnating finished, porous polymer granules such as urea/formaldehyde polymers, polyacrylonitrile and polyesters, having a specific surface area and an advantageous predeterminied absorption/desorption ratio, with the active compounds, for example in the form of their solutions (in a low-boiling solvent) and removing the solvent. Such polymer

granules can be applied in the form of micro-granules with bulk densities of, preferably, 300 g/litre to 600 g/litre, also with the aid of atomisers. Atomising can be effected over extensive treatment areas by means of aircraft.

Granules can also be obtained by compacting the carrier with the active compounds and additives and then comminuting the mixture.

Furthermore, it is possible to add to these agents additives which stabilise the active compound and/or non-ionic, anionic and cationic materials which, for example, improve the adhesion of the active compounds to plants and parts of plants (adhesives and glues) and/or ensure better wettability (wetting agents) and dispersibility (dispersing agents). It is possible to use, for example, the following materials as adhesives: olein/lime mixture, cellulose derivatives (methylcellulose and carboxymethylcellulose), hydroxyethylene glycol ethers of monoalkylphenols and dialkylphenols having 5 to 15 ethylene oxide residues per molecule and 8 to 9 carbon atoms in the alkyl radical, ligninsulphonic acid, its alkali metal salts and alkaline earth metal salts, polyethylene glycol ethers ("Carbowaxes"), fatty alcohol polyglycol ethers having 5 to 20 ethylene oxide residues per molecule and 8 to 18 carbon atoms in the fatty alcohol part, condensation products of ethylene oxide and propylene oxide, polyvinyl-pyrrolidones, polyvinyl alcohols, condensation products of urea/formaldehyde and latex products.

Water-dispersible active compound concentrates, i.e. wettable powders, pastes and emulsion concentrates, are agents which can be diluted with water to any desired concentration. They consist of active compound, carrier, if desired additives which stabilise the active compound, surface-active substances and anti-foaming agents and, if desired, solvents.

The wettable powders and pastes are obtained by mixing and grinding the active compound with dispersing agents and pulverulent carriers in suitable devices until homogeneity is achieved. Examples of carriers are those mentioned above for the solid processing forms. In some cases it is advantageous to use mixtures of different carriers. Examples of dispersing agents which can be used are: condensation products of sulphonated naphthalene and sulphonated naphthalene derivatives with formaldehyde, condensation products of naphthalene or of naphthalene-sulphonic acids with phenol and formaldehyde, and alkali metal salts, ammonium salts and alkaline earth metal salts of ligninsulphinic acid, as well as alkylarylsulphonates, alkali metal salts and alkaline earth metal salts of dibutylnaphthalenesulphonic acid, fatty alcohol sulphates, such as salts of sulphated hexadecanols and heptadecanols, and salts of sulphated fatty alcohol polyethylene glycol ethers, the sodium salt of oleyl methyl tauride, di-tertiary acetylene glycols, dialkyldilauryl ammonium chloride and alkali metal salts and alkaline earth metal salts of fatty acids.

Examples of anti-foaming agents which can be used are silicones.

The active compounds are mixed, ground, sieved and strained with the above mentioned additives, in such a way that the particle size of the solid component does not exceed 0.02 to 0.04 mm in the case of wettable powders and 0.03 mm in the case of pastes. To prepare emulsion concentrates and pastes, dispersing agents, such as have been listed in the preceding sections, organic solvents and water are used. Examples of suitable solvents are the following: alcohols, benzene, xylenes, toluene, dimethylsulphoxide, N,N-dialkylated amides and trialkylamines. The solvents must be virtually odourless and inert towards the active compounds and should not be readily combustible.

Furthermore, the agents according to the invention can be used in the form of solutions. For this purpose, the active compound or several active compounds of the formula I is/are dissolved in suitable organic solvents, solvent mixtures, water or mixtures of organic solvents with water.

The content of active compound in the composition agents described above is between 0.1 and 95%, preferably between 1 and 80%.

Use forms can be diluted down to 0.001%. The amounts used are as a rule 0.1 to 10 kg of active substance/hectare, preferably 0.25 to 5 kg of active substance/hectare.

In terms of their antifungal and antibacterial activity, preferred compounds of formula I are those wherein Z is AY wherein A is O, $R^2$ and $R^3$ are each chlorine and Y is H or $C_1$—$C_6$ alkyl optionally substituted 1—5 chlorine atoms; as well as those wherein A is $NR^7$ wherein $R^7$ is H or $C_1$—$C_6$ alkyl, $R^2$ and $R^3$ are each chlorine and Y has its previous significance.

As intermediates for pyrid-2-on-4-yl derivatives of formula V, preferred compounds of formula I are those wherein A is —$NR^7$ in which $R^7$ is H, $R^2$ and $R^3$ are each chlorine and Y is optionally substituted $C_{1-6}$ alkyl, $C_{2-4}$ alkenyl, $C_{3-6}$ cycloalkyl, benzyl or phenyl.

The following Examples further illustrate the present invention.

Example 1

a) 210 parts of freshly distilled diketene and 30 parts of bis-(t-butylcyclohexyl)perdicarbonate were dissolved in 795 parts of carbon tetrachloride. This solution was added dropwise over 1 hour, with stirring, to 3180 parts of carbon tetrachloride held at reflux temperature in an atmosphere of dry nitrogen. When the addition was complete, stirring at reflux temperature was continued for 1 hour and the solution was then allowed to cool. The excess carbon tetrachloride was distilled off on a rotary evaporator under water pump vacuum at a bath temperature of 30—40°C. The crude product was purified by distillation on a wiped-wall still giving 398 parts of pure 4-chloro-4-(2,2,2-trichloroethyl)oxetan-2-one.

b) 476 Parts of 4-chloro-4-(2,2,2-trichloroethyl)oxetan-2-one were added dropwise over a period of 1

hour to a stirred suspension of 336 parts of sodium hydrogen carbonate in 1500 parts of methanol at 25°C. When the addition was complete, the reaction mixture was stirred at 25°C for 16 hours. The mixture was filtered and the filtrate was concentrated. Distillation of the residue gave 337 parts of methyl 5,5-dichloro-3-oxopent-4-enoate, boiling point 72°C/0.13 millibars.

| | | | |
|---|---|---|---|
| Found | C, 36.45; | H, 3.00; | Cl, 35,85 |
| Calculated for $C_6H_6Cl_2O_3$ | C, 36.57; | H, 3.07; | Cl, 35.99 |

Examples 2—10

When similar reactions to that described in Example 1(b) were carried out using the alcohols and conditions listed in Table 1 the corresponding 5,5-dichloro-3-oxopent-4-enoate esters were obtained.

| Example | Alcohol | Reaction Temp. | Time (hr.) | Distillation Temp./pressure |
|---|---|---|---|---|
| 2 | $C_2H_5OH$ | 80 °C | 4 | 72 °C/0.066 mb |
| 3 | n-$C_4H_9OH$ | 80 °C | 4 | 112–115 °C/0.396 mb |
| 4 | n-$C_8H_{17}OH$ | 80 °C | 4 | 120 °C/0.013 mb |
| 5 | $ClCH_2CH_2OH$ | 80 °C | 4 | 126 °C/0.13 mb |
| 6 | $ClCH_2CH_2CH_2OH$ | 80 °C | 4 | 124 °C/0.066 mb |
| 7 | $Cl_3CCH_2OH$ | 80 °C | 4 | 138 °C/0.39 mb |
| 8 | $CH_2 = CHCH_2OH$ | 80 °C | 6 | 82 °C/0.13 mb |
| 9 | $CH = C–CH_2OH$ | 80 °C | 4 | 98 °C/0.13 mb |
| 10 | 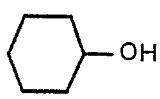 | 80 °C | 6 | 129–130 °C/0.13 mb |

Example 11

a) A mixture of 25.2 parts of diketene, 62.7 parts of bromo-trichloromethane and 1.5 parts of azobisisobutyronitrile dissolved in 978 parts of carbon tetrachloride was irradiated by UV light at room temperature for 5 hours. The mixture was evaporated at the water pump and the product, 4-bromo-4-(2,2,2-trichloroethyl)oxetan-2-one, was obtained as a yellow oil which crystallised on cooling. Recrystallisation of this material three times from light petroleum ether (b.p. 40—60° gave white needles m.p. 55—56°C.

| | | |
|---|---|---|
| Found | C, 21.09; | H, 1.53 |
| Calculated for $C_5H_4BrCl_3O_2$ | C, 21.27; | H, 1.43 |

b) To a stirred solution of 85 parts of 4-bromo-4-(2,2,2-trichloroethyl)-oxetan-2-one in 200 parts of carbon tetrachloride at 70° was added 100 parts of methanol, dropwise, over 15 minutes. The solution was heated at reflux temperature for 1 hour, cooled and concentrated.

Distillation of the residue gave methyl 5,5-dichloro-3-oxopent-4-enoate, b.p. 72°/0.13 millibars, identical to that obtained in Example 1.

Example 12

30 Parts of 4-chloro-4-(2,2,2-trichloroethyl)oxetan-2-one was added dropwise, over 30 miinutes, to 500 parts of methanol maintained at reflux temperature. The solution was refluxed for 2.5 hours, cooled and

concentrated. The residue was distilled to give methyl 5,5-dichloro-3-oxopent-4-enoate, b.p. 75°/0.26 millibars, identical with the product obtained in Example 1.

Example 13

a) A mixture of 8.4 parts of diketene, 33.1 parts of carbon tetrabromide, 0.5 parts of bis-(t-butylcyclohexyl)-perdicarbonate and 326 parts of carbon tetrachloride was heated at 60°C for 2 hours, then evaporated at the water pump. The residue was recrystallised twice from light petroleum ether (b.p. 40—60°C) and the product, 4-bromo-4-(2,2,2-tribromoethyl)oxetan-2-one, obtained as white needles m.p. 71—73°C.

| | | | |
|---|---|---|---|
| Found | C, 14.75; | H, 0.97; | Br, 76.20 |
| Calculated for $C_5H_4Br_4O_2$ | C, 14.45; | H, 0.97; | Br, 76.89 |

b) 400 Parts of methanol were added dropwise to a stirred suspension of 40.3 parts of 4-bromo-4-(2,2,2-tribromoethyl)oxetan-2-one and 19.2 parts of sodium hydrogen carbonate in 800 parts of carbon tetrachloride. The reaction mixture was heated at reflux for 3 hrs., cooled and filtered. The filtrate was concentrated and the pale brown residual oil was distilled to give methyl 5,5-dibromo-3-oxopent-4-enoate b.p. 80—86°C/0.026 millibars.

| | | |
|---|---|---|
| Found | C, 25.37; | H, 2.29 |
| Calculated for $C_6H_6Br_2O_3$ | C, 25.20; | H, 2.12 |

Example 14

56.4 Parts of phenol were added to a suspension of 32.4 parts of sodium methoxide in 500 parts of methylene chloride and the resulting mixture stirred at room temperature for ten minutes. 47.6 parts of 4-chloro-4-(2,2,2-trichloroethyl)oxetan-2-one in 50 parts of methylene chloride were added dropwise keeping the reaction temperature at 20°C by external cooling. The mixture was left stirring overnight then washed with water, dried ($MgSO_4$) and evaporated to an oil consisting mainly of phenol and product. Phenol was distilled from the oil by passing it down a wiped wall still at 90°C/0.01 millibars. The residue was recrystallised from hexane to give phenyl 5,5-dichloro-3-oxopent-4-enoate, m.p. 54—56°C.

Example 15

A solution of 1.0 part of 4-chloro-4-(2,2,2-trichloroethyl)oxetan-2-one in 10 parts of concentrated sulphuric acid was stirred at 25°C for 2 hours. The reaction mixture was diluted with water and extracted with ethyl acetate. The organic extracts were dried ($MgSO_4$) and concentrated to give 5,5-dichloro-3-oxopent-4-enoic acid having m.p. 109—111°C.

Example 16

A mixture of 1.83 parts of 5,5-dichloro-3-oxopent-4-enoic acid, 2.08 parts of phosphorus pentachloride and 30 parts of dry carbon tetrachloride was stirred at ambient temperature for 2 hrs. After this time, the clear solution was evaporated in vacuo at ambient temperature. Dry toluene (25 parts) was added and the solution was again evaporated to give 1.9 parts of 5,5-dichloro-3-oxopent-4-enoyl chloride.

IR ($CCl_4$) 1790 $cm^{-1}$ (C=O absorption)

NMR (δ, $CCl_4$) 4.05 (s, $—CH_2—$, keto form); 5.75 (s, = CH—COCl, enol form); 6.35 (s, $Cl_2C$ = CH—, enol form); 6.75 (s, $Cl_2C$ = CH— keto form); 11.2 (br.s. —C(OH)—, enol form).

Example 17

When the procedure described in Example 15 was repeated but using, as starting material 4-bromo-4-(2,2,2-tribromoethyl)oxetan-2-one, there was obtained 5,5-dibromo-3-oxopent-4-enoic acid, having m.p. 86—89°C.

Example 18

A solution of 5.9 parts of isopropylamine in 50 parts of methylene chloride was added dropwise at 25°C to a stirred suspension of 23.8 parts of 4-chloro-4(2,2,2-trichloroethyl)oxetan-2-one, 17.6 parts of sodium hydrogen carbonate and 100 parts of methylene chloride. The temperature of the reaction mixture was kept below 35°C during the addition with the aid of an ice-water cooling bath. The reaction mixture was stirred at room temperature for 16 hrs. and filtered.

Concentration of the filtrate afforded 21.1 parts of N-isopropyl-5,5-dichloro-3-oxopent-4-enamide having m.p. 74—75°C. Recrystallisation from absolute ethanol raised m.p. to 80—81°C.

| | | | | |
|---|---|---|---|---|
| Found | C, 43.00; | H, 4.97; | Cl, 31.46; | N, 5.92 |
| Calculated for $C_8H_{11}Cl_2NO_3$ | C, 42.88; | H, 4.95; | Cl, 31.64; | N, 6.25 |

Examples 19—41

When similar reactions to that described in Example 18 were carried out using the amines listed in Table 2, the corresponding 5,5-dichloro-3-oxopent-4-enamides were obtained.

TABLE 2

Preparation of 5,5-Dichloro-3-oxopent-4-enamides

| Example | Amine | Solvent | Product m.p. |
|---|---|---|---|
| 19 | $n\text{-}C_3H_7NH_2$ | Diethylether | 69–70 °C |
| 20 | $HOCH_2CH_2NH_2$ | $CH_2Cl_2$ | oil * |
| 21 | $HO(CH_2)_6CH_2NH_2$ | $CH_2Cl_2$ | oil ** |
| 22 | $cyclo\text{-}C_3H_5NH_2$ | $CH_2Cl_2$ | 80–81 °C |
| 23 | $tert\text{-}C_4H_9NH_2$ | $CH_2Cl_2$ | 79–80 °C |
| 24 | $n\text{-}C_8H_{17}NH_2$ | $CH_2Cl_2$ | 56–57 °C |
| 25 | $CH_3(CH_2)_{14}CH_2NH_2$ | $CH_2Cl_2$ | 73–74 °C |
| 26 | $CH_3(CH_2)_{16}CH_2NH_2$ | $CH_2Cl_2$ | 76–79 °C |
| 27 | $C_2H_5O_2CCH_2NH_2$ | $CH_2Cl_2$ | 87–89 °C |
| 28 | $C_6H_5CH_2NH_2$ | $CH_2Cl_2$ | 88–89 °C |
| 29 | $(C_6H_5)_2CHNH_2$ | $CH_2Cl_2$ | 148–149 °C |
| 30 | 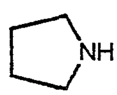 | $CH_2Cl_2$ | 84–87 °C |
| 31 | 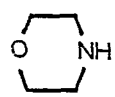 | $CH_2Cl_2$ | 119–120 °C |
| 32 | 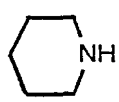 | Diethylether | 57–60 °C |
| 33 | $C_6H_5NH_2$ | $CH_2Cl_2$ | 118–119 °C |
| 34 | 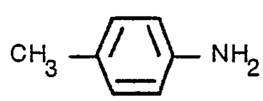 | $CH_2Cl_2$ | 117–118 °C |
| 35 | 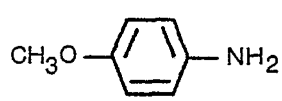 | $CH_2Cl_2$ | 101–103 °C |
| 36 | 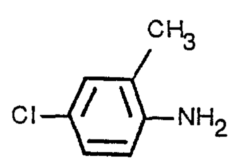 | $CH_2Cl_2$ | 102–103 °C |

TABLE 2 (Continued)

Preparation of 5,5-Dichloro-3-oxopent-4-enamides

| Example | Amine | Solvent | Product m.p. |
|---|---|---|---|
| 37 | 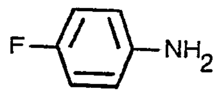 | $CH_2Cl_2$ | 97—98°C |
| 38 | $CH_2=CHCH_2NH_2$ | $CH_2Cl_2$ | 70—71°C |
| 39 | 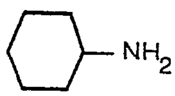 | $CH_2Cl_2$ | 99—100°C |
| 40 | $(CH_2=CHCH_2)_2NH$ | $CH_2Cl_2$ | oil*** |
| 41 | 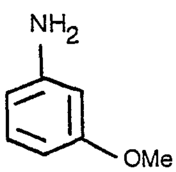 | $CH_2Cl_2$ | 77—78°C |

| | | | |
|---|---|---|---|
| *Found | C, 37.22; | H, 4.16; | N, 6.21 |
| Calculated for $C_7H_9Cl_2NO_3$ | C, 37.19; | H, 4.01; | N, 6.19 |

**IR: $\gamma_{max}$ (C=O) 1700, (C=O) 1630, (C=C) 1550 $cm^{-1}$

| | | | |
|---|---|---|---|
| ***Found | C, 50.35; | H, 5.05; | N, 5.68 |
| Calculated for $C_{11}H_{13}Cl_2NO_2$ | C, 50.40; | H, 5.00; | N, 5.34 |

Example 42

A solution of 116 parts of concentrated ammonia (SG 0.88) in 100 parts of water was added dropwise to a stirred solution of 238 parts of 4-chloro-4(2,2,2-trichloroethyl)oxetan-2-one in 500 parts of dioxan keeping the reaction temperature below 35°C with the aid of an ice-water bath. The reaction mixture was stirred for 4 hours and extracted with chloroform. The chloroform extracts were washed with brine, dried ($MgSO_4$), filtered and concentrated to give 120 parts of 5,5-dichloro-3-oxopent-4-enamide having m.p. 109—112°C. Recrystallisation from a mixture of ethyl acetate and 60—80°C petroleum ether raised the m.p. to 112—114°C.

| | | | |
|---|---|---|---|
| Found | C, 32.87; | H, 2.69; | N, 7.65 |
| Calculated for $C_5H_5Cl_2NO_2$ | C, 33.06; | H, 2.73; | N, 7.65 |

Example 43 and 44

When similar reactions to that described in Example 42 were carried out using the amines listed in Table 3 the corresponding 5,5-dichloro-3-oxopent-4-enamides were obtained.

TABLE 3

Preparation of 5,5-Dichloro-3-oxopent-4-enamides

| Example | Amine | Product m.p. |
|---|---|---|
| 43 | $CH_3NH_2$ | 84—85°C |
| 44 | $(CH_3)_2NH$ | 105—106°C |

Example 45

A mixture of 180 parts of 4-chloro-4(2,2,2-trichloroethyl)oxetan-2-one, 89.3 parts of 2,6-dimethylaniline, 61.5 parts of sodium acetate in 1000 parts of glacial acetic acid was stirred at 25°C for 48 hours and filtered. The filtrate was concentrated to dryness and the solid residue was recrystallised from ethanol to give N-(2,6-dimethylphenyl)-5,5-dichloro-3-oxopent-4-enamide having m.p. 109—110°C.

| | | | | |
|---|---|---|---|---|
| Found | C, 54.14; | H, 4.55; | Cl, 25.15; | N, 4.75 |
| Calculated for $C_{13}H_{13}Cl_2NO_3$ | C, 54.57; | H, 4.89; | Cl, 24.78; | N, 4.89 |

Example 46

A solution of 7.5 parts of glycine in 50 parts of acetone and 100 parts of water was added dropwise at 25°C to a stirred suspension of 24 parts of 4-chloro-4-(2,2,2-trichloroethyl)oxetan-2-one, 17 parts of sodium hydrogen carbonate and 200 parts of acetone. The reaction mixture was stirred for 16 hours and filtered. Acidification of the filtrate with 2N hydrochloric acid followed by extraction with chloroform gave, after drying ($MgSO_4$) and concentrating, N-(carboxymethyl)-5,5-dichloro-3-oxopent-4-enamide having m.p. 116—119°. Recrystallisation from ethyl acetate raised the m.p. to 120—122°C.

| | | | |
|---|---|---|---|
| Found | C, 35.08; | H, 2.99; | N, 5.68 |
| Calculated for $C_7H_7Cl_2NO_4$ | C, 35.00; | H, 2.92; | N, 5.83 |

Example 47—49

When similar reactions were carried out using the amines listed in Table 4 the corresponding 5,5-dichloro-3-oxopent-4-enamides were obtained.

TABLE 4

Preparation of 5,5-Dichloro-3-oxopent-4-enamides

| Example | Amine | Product m.p. |
|---|---|---|
| 47 | 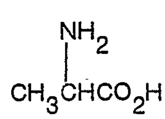 | 131—132°C |
| 48 | 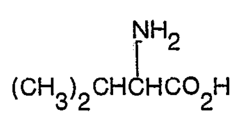 | 84—88°C |
| 49 | $HO_2CCH_2CH_2NH_2$ | 94—96°C |

Example 50

When a similar reaction to that described in Example 18 was carried out using 4-bromo-4-(2,2,2-tribromoethyl)-oxetan-2-one and aniline, N-phenyl-5,5-dibromo-3-oxopent-4-enamide, m.p. 136—137°C was formed.

Example 51

A mixture of 25.8 parts of ethyldi-isopropylamine and 11.0 parts of thiophenol in 100 parts of chloroform were added dropwise at room temperature to 23.4 parts of 4-chloro-4-(2,2,2-trichloroethyl)oxetan-2-one in 100 parts of chloroform. The reaction mixture was left for 18 hours at ambient temperature, then evaporated. The semi-crystalline solid was triturated with a little methanol, the solid was collected and crystallised from methanol to give phenyl 5,5-dichloro-3-oxopent-4-enthiolate m.p. 66—69°C.

| | | |
|---|---|---|
| Found | C, 47.97; | H, 3.00 |
| Calculated for $C_{11}H_8Cl_2O_2S$ | C, 48.02; | H, 2.93 |

Example 52

In a similar manner to Example 51 using methanethiol instead of thiophenol there was obtained methyl 5,5-dichloro-3-oxopent-4-enthiolate.

Example 53

In a similar manner to Example 51 using ethyl thioglycolate instead of thiophenol there was obtained ethoxycarbonylmethyl 5,5-dichloro-3-oxopent-4-enthiolate.

Example 54

The fungicidal and bactericidal activity of compounds of formula I was examined as follows:

Activity against Phytophthora infestans on tomato plants

a) *Residual-curative activity*

Three week old tomato plants were infected with a suspension of fungal sporangia. After incubation for 22 hours in a humidity chamber at 90—100% relative humidity at 20°C the plants were sprayed with a broth containing 0.06% of the compound to be tested. After drying of the spray-coating the plants were returned to the humidity chamber. Evaluation of fungal attack was made 5 days after infection.

b) *Systemic activity*

Three week old tomato plants were watered with broth containing the test compound to give a concentration thereof of 0.006% with respect to soil volume. Care was taken to prevent contact between the broth and the aerial plant parts. After 48 hours the plants were infected with a suspension of fungal sporangia. Evaluation of fungal attack was made after 5 days incubation of the infected plants at 90—100% relative humidity and 20°C.

Activity against Xanthomonas oryzae on rice

a) *Residual-protective activity*

Three week old rice plants of the variety "Caloro" or "S6" were sprayed with a broth containing 0.06% of the compound to be tested.

After drying for one day the plants were placed in a controlled climate room at 24° and 75—85% relative humidity and infected by removing the leaf tips with scissors previously dipped in a suspension of Xanthomonas oryzae. After 10 days of incubation in the same room diseased leaves withered, curled and become necrotic. The extent of these symptoms was used to evaluate the activity of the test compound.

b) *Systemic activity*

Three week old rice plants of the variety "Caloro" or "S6" were watered with a broth containing the test compound to give a concentration thereof of 0.006% with respect to soil volume. Care was taken to prevent contact between the broth and the aerial plant parts. Three days after treatment the plants were placed in a controlled climate room at 24°C and 75—85% relative humidity and infected by removing the leaf tips with scissors previously dipped in a suspension of Xanthomonas oryzae. After 10 days of incubation in the same room diseased leaves withered, curled and became necrotic. The extent of these symptoms was used to evaluate the activity of the test compound.

Activity against Helminthosporium gramineum

Wheat grains were contaminated with a sporal suspension of the fungus, dried and dressed with a broth containing the test substance to give a concentration thereof of 0.06% with respect to the weight of seed grains. After 10 days the grains were placed on suitable agar dishes and after two days the development of fungal colonies round the grains was determined.

Activity against Cercospora arachidicola on ground nut plants

10—15 cm high ground-nut plants were sprayed with a broth containing 0.02% of the compound to be tested and after 48 hours infected with a suspension of fungal conidia. The infected plants were incubated for 72 hours at ca. 21°C and high humidity and then placed in a greenhouse until the appearance of typical leafspots on the control plants. Evaluation of the fungal attack took place 12 days after infection on the basis of the number and size of leaf spots.

The compounds of the Formula I exhibited a good fungicidal and bactericidal activity. The following compounds, amongst others, inhibited the fungal or bacterial attack in comparison with untreated but infected controls.

against Phytophthora infestans: compound of Example 34
against Xanthomonas oryzae: compound of Example 48
against Helminthosporium gramineum: compound of Example 1b
against Cercospora arachidicola: compound of Example 35

Use Example 55

In a similar manner to that described in Example 54 anti-fungal activity against Botrytis infections on vicia, Erysiphe graminis infections on barley, Fusarium infections on rye seed, and Piricularia infections on maize was evaluated. The compounds of the Formula I exhibited a good fungicidal activity. The following compounds, amongst others, inhibited the fungal attack in comparison with untreated but infected controls:

against Botrytis: compound of Example 35
against Erysiphe graminis: compound of Example 7
against Fusarium: compounds of Examples 7 and 13
against Piricularia: compound of Example 5

**Claims**

1. A process for the production of a compound having the formula:

$$R^2R^3C = CHCOCH_2COZ \qquad I$$

wherein $R^2$ is fluorine, chlorine, or bromine; $R^3$ is hydrogen, fluorine, chlorine or bromine, or a group of formula $—CR^4R^5R^6$ wherein $R^4$,$R^5$ and $R^6$ are fluorine, chlorine or bromine; and Z is —AY or $X^1$, wherein A is —O—, —S— or $—NR^7$ wherein $R^7$ is hydrogen, $C_{1-10}$-straight- or branch chain alkyl or $C_{3-4}$ straight or branch chain alkenyl, Y is hydrogen, $C_{1-18}$-straight or branch chain alkyl optionally interrupted by one to five oxygen atoms and optionally substituted by one to five chlorine or bromine atoms, or by a group having the formula $—OR^8$ or the formula $—CO_2R^9$ wherein $R^8$ is hydrogen, $C_{3-12}$-cycloalkyl, straight- or branch chain $C_{3-18}$-alkenyl, $C_{6-10}$-aryl or $C_{7-13}$-aralkyl and $R^9$ is hydrogen, $C_{1-18}$-straight- or branch chain alkyl or straight- or branch chain $C_{3-18}$-alkenyl, or Y is $C_{3-18}$ straight or branch chain alkenyl, $C_{3-18}$ straight- or branch chain alkynyl, $C_{3-12}$-cycloalkyl, $C_{7-13}$-aralkyl, or $C_{6-10}$-aryl which is optionally substituted by one to three $C_{1-9}$ straight- or branch chain alkyl, halogen, nitro, $CF_3$, or $SO_3H$, or groups $—OR^{10}$ wherein $R^{10}$ is hydrogen or $C_{1-4}$ straight- or branch chain alkyl, provided that the total number of carbon atoms in the group —AY is from 0 to 20; or $R^7$ and Y together may form a $C_{2-7}$ polymethylene chain optionally substituted by one or two $C_{1-4}$ alkyl groups and optionally interrupted by —O—, —S— or —NR'' groups wherein R'' is $C_{1-4}$ straight or branch chain alkyl; and $X^1$ is Cl or Br; comprising (a) reacting, optionally in the presence of a solvent and an inorganic or organic acid-binding agent, a compound having the formula II:

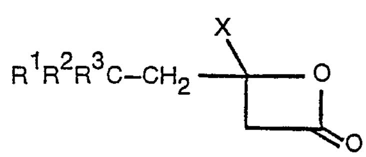

II

wherein $R^2$ and $R^3$ have their previous significance; $R^1$ is fluorine, chlorine or bromine; and X is chlorine, bromine, or iodine with the proviso that, when X is chlorine, none of $R^1$, $R^2$, $R^3$, $R^4$, $R^5$ and $R^6$ can be bromine; with a compound of formula H—Z (III) wherein Z has its previous significance; and (b) when Z is —AY and —AY is —OH, the product of step a) is optionally reacted with a compound (IV) capable of converting a carboxyl group into the corresponding carboxylic acid chloride or -bromide, thereby producing a compound of formula I wherein Z is- $X^1$.

2. A process according to claim 1 wherein, in step a), the solvent is either one which is inert under the reaction conditions, or is an excess of one of the reagents.

3. A process according to claim 1 or 2 wherein the reaction temperature in step a) is from −20° to 150°C.

4. A process according to claim 1, wherein A is O and a lactone of formula II is reacted with an excess of an alcohol of formula III in the presence of an excess of acid binding agent.

5. A process according to claim 1 wherein A is $NR^7$ wherein $R^7$ is as defined in claim 1, and equimolar quantities of a lactone of formula II and an amine of formula III are stirred together in an inert solvent in the presence of an excess of acid binding agent.

6. A process according to claim 1 wherein A is $NR^7$ wherein $R^7$ is as defined in claim 1 and a lactone of formula II is reacted with an amine of formula III in aqueous dioxan, excess amine being used as acid binding agent.

7. A process according to any of the preceding claims wherein a lactone of formula II is used in which $R^1$, $R^2$, $R^3$ and X are each chlorine or bromine.

8. A process according to claim 7 wherein $R^1$, $R^2$, $R^3$ and X are each chlorine.

9. An antifungal and antibacterial composition comprising, as active ingredient, a compound of formula I as defined in claim 1.

10. An antimicrobial composition according to claim 9 comprising an amount of a compound of formula I, as defined in claim 1, which is effective against the growth of bacteria and fungi which are pathogenic to plants.

11. A composition according to claim 9 wherein A is O or $NR^7$ wherein $R^7$ is as defined in claim 1.

12. A composition according to claim 11 wherein $R^2$ and $R^3$ are each chlorine.

13. A composition according to claim 12 wherein A is O and Y is H or alkyl having from 1 to 6 carbon atoms and optionally substituted by from 1 to 5 chlorine atoms.

14. A composition according to claim 12 wherein A is $NR^7$ wherein $R^7$ is H or alkyl having from 1 to 6 carbon atoms.

15. Compounds of the formula:

$$R^2R^3C = CHCOCH_2COZ \qquad IA$$

wherein $R^2$ is fluorine, chlorine, or bromine; $R^3$ is hydrogen, fluorine, chlorine or bromine, or a group of formula $—CR^4R^5R^6$ wherein $R^4$, $R^5$ and $R^6$ are fluorine, chlorine or bromine; Z is —AY or — $X^1$ A is —O—, —S— or $—NR^7$ wherein $R^7$ is hydrogen, $C_1—C_{10}$ straight or branch chain alkyl or $C_3—C_4$ straight or branch chain alkenyl and Y is hydrogen, $C_1—C_{18}$ straight or branch chain alkyl optionally interrupted by one to five oxygen atoms and optionally substituted by one to five chlorine or bromine atoms, or by a group having the formula $—OR^8$ or the formula $—CO_2R^9$ wherein $R^8$ is H, $C_3—C_{12}$ cycloalkyl, $C_3—C_{18}$ straight- or branch chain alkenyl, $C_6—C_{10}$ aryl or $C_7—C_{13}$ aralkyl and $R^9$ is hydrogen, $C_1—C_{18}$ straight or branch chain alkyl or $C_3—C_{18}$ straight- or branch chain alkenyl, or Y is $C_3—C_{18}$ straight or branch chain alkenyl, $C_3—C_{18}$ straight or branch chain alkynyl, $C_3—C_{12}$ cycloalkyl, $C_7—C_{13}$ aralkyl or $C_6—C_{10}$ aryl which is optionally substituted by one to three $C_1—C_9$ straight or branch chain alkyl, halogen, nitro, $CF_3$, $SO_3H$ or groups $OR^{10}$ wherein $R^{10}$ is hydrogen or $C_1—C_4$ straight or branch chain alkyl; provided that the total number of carbon atoms in the group —AY is from 0 to 20; or $R^7$ and Y together may form a $C_2—C_7$ polymethylene chain optionally substituted by one or two $C_1—C_4$ alkyl groups and optionally interrupted by O, S or $NR^{11}$ groups where $R^{11}$ is $C_1—C_4$ striaght or branch chain alkyl; and $X^1$ is Cl or Br; except that, when $R^2$ and $R^3$ are each chlorine and A is —O—, then Y is other than hydrogen or ethyl.

16. Compounds according to claim 15 wherein A is —O— or $—NR^7$ wherein $R^7$ is as defined in claim 1.

17. Compounds according to claim 16 wherein $R^2$ and $R^3$ are each chlorine.

18. Compounds according to claim 17 wherein A is $NR^7$ wherein $R^7$ is H or alkyl having 1 to 6 carbon atoms.

19. Compounds according to claim 17 wherein A is $NR^7$ where $R^7$ is H, $R^2$ and $R^3$ are Cl and Y is optionally substituted $C_{1-6}$ alkyl, $C_{2-4}$ alkenyl, $C_{3-6}$ cycloalkyl, benzyl or phenyl.

20. Compounds according to claim 17 wherein A is —O— and Y is methyl or $C_{3-6}$ alkyl.

**Patentansprüche**

1. Verfahren zur Herstellung einer Verbindung der Formel I

$$R^2R^3C=CHCOCH_2COZ \qquad I$$

worin $R^2$ Fluor, Chlor oder Brom ist; $R^3$ Wasserstoff, Fluor, Chlor oder Brom ist oder eine Gruppe der Formel $—CR^4R^5R^6$ bedeutet, worin $R^4$, $R^5$ und $R^6$ Fluor, Chlor oder Brom sind; und Z entweder —AY oder $X^1$ darstellt, wobei A wahlweise —O—, —S— oder $—NR^7$ bedeutet, mit $R^7$ = Wasserstoff, geradkettiges oder verzweigtes $C_1—C_{10}$-Alkyl oder geradkettiges oder verzweigtes $C_3—C_4$-Alkenyl; während Y Wasserstoff, unverzweigtes oder verzweigtes $C_1—C_{18}$-Alkyl bedeutet, das wahlweise durch 1 bis 5 Sauerstoffatome unterbrochen und wahlweise durch 1 bis 5 Chlor- oder Bromatome oder durch eine Gruppe der Formel $—OR^8$ oder der Formel $—CO_2R^9$ substituiert sein kann, in der $R^8$ Wasserstoff, $C_3—C_{12}$-Cycloalkyl, unverzweigtes oder verzweigtes $C_3—C_{18}$-Alkenyl, $C_6—C_{10}$-Aryl oder $C_7—C_{13}$-Aralkyl ist; $R^9$ Wasserstoff, unverzweigtes oder verzweigtes $C_1—C_{18}$-Alkyl oder unverzweigtes oder verzweigtes $C_3—C_{18}$-Alkenyl ist; oder worin Y unverzweigtes oder verzweigtes $C_3—C_{18}$-Alkenyl, unverzweigtes oder verzweigtes $C_3—C_{18}$-Alkinyl, $C_3—C_{12}$-Cycloalkyl, $C_7—C_{13}$-Aralkyl oder $C_6—C_{10}$-Aryl bedeutet, wobei Aryl wahlweise durch ein bis drei Substituenten $C_1—C_9$-Alkyl, geradkettig oder verzweigt, Halogen, Nitro, $CF_3$, $—SO_3H$ oder durch $—OR^{10}$

substituiert sein kann, wobei $R^{10}$ Wasserstoff oder geradkettiges oder verzweigtes $C_1$—$C_4$ Alkyl ist; mit der Massgabe, dass die Gesamtzahl der C-Atome in der Gruppe —AY Null bis zwanzig beträgt; oder worin $R^7$ und Y zusammen eine $C_2$—$C_7$ Polymethylenkette bilden, die wahlweise durch eine oder zwei $C_1$—$C_4$-Alkylgruppen substituiert und wahlweise durch —O—, —S— oder —NR″— unterbrochen sein kann, wobei R″ geradkettiges oder verzweigtes $C_1$—$C_4$-Alkyl ist; und worin $X^1$ Chlor oder Brom bedeutet; dadurch gekennzeichnet, dass

a) eine Verbindung der Formel II

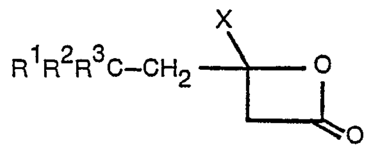

II

wahlweise in Gegenwart eines Lösungsmittel und eines anorganischen oder organischen säurebindenden Mittels mit einer Verbindung der Formel III

$$H—Z \qquad III$$

worin Z wie oben definiert ist, zur Reaktion gebracht wird; wobei $R^2$ und $R^3$ die obengenannten Bedeutungen haben, $R^1$ Fluor, Chlor oder Brom ist und X Chlor, Brom oder Jod ist, mit der Massgabe, dass keiner der Substituenten $R^1$, $R^2$, $R^3$, $R^4$, $R^5$ und $R^6$ Brom sein kann, wenn X Chlor ist; und

b) wenn Z die Bedeutung —AY hat und —AY eine OH-Gruppe ist, das unter a) erhaltene Produkt wahlweise mit einer Verbindung IV umgesetzt wird, die befähigt ist, eine Carboxylgruppe in ein Carbonsäurechlorid oder -bromid umzuwandeln, wobei eine Verbindung der Formel I erzeugt wird, in der Z dem Substituenten $X^1$ entspricht.

2. Verfahren nach Anspruch 1, worin in Stufe a) das Lösungsmittel ein unter den Reaktionsbedingungen inertes Lösungsmittel oder der Ueberschuss einer der Reaktanden ist.

3. Verfahren nach Anspruch 1 oder 2, worin die Reaktionstemperaturen in Stufe a) bei −20°C bis +150°C liegen.

4. Verfahren nach Anspruch 1, worin A Sauerstoff ist und ein Lacton der Formel II mit einem Ueberschuss eines Alkohols der Formel III in Gegenwart eines Ueberschusses an säurebindenden Mittel umgesetzt wird.

5. Verfahren nach Anspruch 1, worin A die Bedeutung $—NR^7—$ hat und $R^7$ die im Anspruch 1 gegebene Bedeutung hat, und wobei äquimolare Mengen eines Lactons der Formel II und eines Amins der Formel III zusammen in einem inerten Lösungsmittel in Gegenwart eines Ueberschusses an säurebindendem Mittel gerührt werden.

6. Verfahren nach Anspruch 1, worin A $—NR^7—$ ist und $R^7$ die im Anspruch 1 angegebene Bedeutung hat, und wobei ein Lacton der Formel II mit einem Amin der Formel III in wässrigem Dioxan umgesetzt wird, wobei als säurebindendes Mittel ein Ueberschuss an Amin verwendet wird.

7. Verfahren nach einem der vorstehenden Ansprüche, dadurch gekennzeichnet, dass man ein Lacton der Formel II verwendet, worin $R^1$, $R^2$, $R^3$ und X je Chlor oder Brom bedeuten.

8. Verfahren nach Anspruch 7, dadurch gekennzeichnet, dass $R^1$, $R^2$, $R^3$ und X je Chlor bedeuten.

9. Mittel zur Bekämpfung von Pilzen und Bakterien, das als Wirkstoff eine Verbindung der Formel I nach Anspruch 1 enthält.

10. Antimikrobielles Mittel nach Anspruch 9, das eine zur Bekämpfung von pflanzenschädigenden Bakterien und Pilzen ausreichende Menge an einer Verbindung der Formel I nach Anspruch 1 enthält.

11. Mittel nach Anspruch 9, worin A Sauerstoff oder $—NR^7—$ darstellt und $R^7$ die im Anspruch 1 angegebene Bedeutung hat.

12. Mittel nach Anspruch 11, worin $R^2$ und $R^3$ je Chlor darstellen.

13. Mittel nach Anspruch 12, worin A Sauerstoff und Y Wasserstoff oder $C_1$—$C_6$-Alkyl, das durch 1 bis 5 Chloratome substituiert sein kann, darstellen.

14. Mittel nach Anspruch 12, worin A $—NR^7—$ ist und $R^7$ Wasserstoff oder Alkyl mit 1 bis 6 C-Atomen darstellt.

15. Verbindungen der Formel IA

$$R^2R^3C{=}CHCOCH_2COZ \qquad (IA),$$

worin $R^2$ Fluor, Chlor oder Brom ist, $R^3$ Wasserstoff, Fluor, Chlor, Brom oder eine Gruppe $—CR^4R^5R^6$ ist, in der $R^4$, $R^5$ und $R^6$ Fluor, Chlor oder Brom, sind, Z den Rest —AY oder $X^1$ bedeutet und A Sauerstoff, Schwefel oder $—NR^7—$ darstellt, worin $R^7$ Wasserstoff, geradkettiges oder verzweigtes $C_1$—$C_{10}$-Alkyl oder geradkettiges oder verzweigtes $C_3$—$C_4$-Alkenyl und Y Wasserstoff, geradkettiges oder verzweigtes $C_1$—$C_{18}$-Alkyl, das durch 1 bis 5 Sauerstoffatome unterbrochen und durch 1 bis 5 Chlor- oder Bromatome oder durch eine Gruppe $—COR^8$ oder $—COOR^9$ substituiert sein kann, worin $R^8$ Wasserstoff, $C_3$—$C_{12}$-Cycloalkyl, $C_3$—$C_{18}$ geradkettig oder verzweigtes Alkenyl, $C_6$—$C_{10}$-Aryl oder $C_7$—$C_{13}$-Aralkyl bedeutet und $R^9$

Wasserstoff, geradkettig oder verzweigtes $C_1$—$C_{18}$-Alkyl oder geradkettig oder verzweigtes $C_3$—$C_{18}$-Alkenyl darstellt, und Y geradkettiges oder verzweigtes $C_3$—$C_{18}$-Alkenyl, geradkettiges oder verzweigtes $C_3$—$C_{18}$-Alkynyl, $C_3$—$C_{12}$-Cycloalkyl, $C_7$—$C_{13}$-Aralkyl oder $C_6$—$C_{10}$-Aryl, das ein- bis dreifach durch geradkettiges oder verzweigtes $C_1$—$C_9$-Alkyl, Halogen, Nitro, $CF_3$, —$SO_3H$ oder —$OR^{10}$ substituiert sein kann, bedeutet, wobei $R^{10}$ Wasserstoff oder geradkettiges oder verzweigtes $C_1$—$C_4$-Alkyl ist; und wobei die C-Zahl der Gruppe —AY Null bis 20 beträgt, oder $R^7$ und Y zusammen eine $C_2$—$C_7$-Polymethylenkette darstellen, die durch eine oder zwei $C_1$—$C_4$-Alkylgruppen substituiert und durch —O—, —S— oder —$NR^{11}$— unterbrochen sein kann, wobei $R^{11}$ geradkettiges oder verzweigtes $C_1$—$C_4$-Alkyl bedeutet, und $X^1$ Chlor oder Brom ist, mit der Massgabe, dass Y, wenn $R^2$ und $R^3$ je Chlor und A Sauerstoff darstellen, ungleich Wasserstoff oder Aethyl ist.

16. Verbindungen nach Anspruch 15, worin A Sauerstoff oder —$NR^7$ ist und $R^7$ die im Anspruch 1 angegebene Bedeutung hat.

17. Verbindungen nach Anspruch 16, worin $R^2$ und $R^3$ je Chlor darstellen.

18. Verbindungen nach Anspruch 17, worin A —$NR^7$— und $R^7$ Wasserstoff oder $C_1$—$C_6$-Alkyl bedeuten.

19. Verbindungen nach Anspruch 17, worin A die Bedeutung —$NR^7$— hat, mit $R^7$ = Wasserstoff, $R^2$ und $R^3$ je Chlor sind und Y wahlweise substituiertes $C_1$—$C_6$-Alkyl, $C_2$—$C_4$-Alkenyl, $C_3$—$C_6$-Cycloalkyl, Benzyl oder Phenyl ist.

20. Verbindungen nach Anspruch 17, worin A Sauerstoff ist und Y Methyl oder $C_3$—$C_6$-Alkyl.

**Revendications**

1. Un procédé pour la production d'un composé répondant à la formule:

$$R^2R^3C{=}CHCOCH_2COZ \qquad I$$

dans laquelle $R^2$ est un fluor, un chlore ou un brome, $R^3$ est un hydrogène, un fluor, un chlore ou un brome, ou un groupe de formule —$CR^4R^5R^6$ où $R^4$, $R^5$ et $R^6$ sont un fluor, un chlore ou un brome; et Z est —AY ou $X^1$, où A est —O—, —S— ou —$NR^7$— où $R^7$ est un hydrogène, un alkyle à chaîne droite ou ramifiée en $C_{1-10}$ ou un alcényle à chaîne droite ou ramifiée en $C_{3-4}$, Y est un hydrogène, un alkyle à chaîne droite ou ramifiée en $C_{1-18}$ éventuellement interrompu par 1 à 5 atomes d'oxygène et éventuellement substitué par 1 à 5 atomes de chlor ou de brome, ou par un groupe de formule —$OR^8$ ou de formule —$CO_2R^9$ où $R^8$ est un hydrogène, un cycloalkyle en $C_{3-12}$, un alcényle à chaîne droite ou ramifiée en $C_{3-18}$, un aryle en $C_{6-10}$ ou un aralkyle en $C_{7-13}$ et $R^9$ est un hydrogène, un alkyle à chaîne droite ou ramifiée en $C_{1-18}$ ou un alcényle à chaîne droite ou ramifiée en $C_{3-18}$, ou Y est un alcényle à chaîne droite ou ramifiée en $C_{3-18}$, un alcynyle à chaîne droite ou ramifiée en $C_{3-18}$, un cycloalkyle en $C_{3-12}$, un aralkyle en $C_{7-13}$ ou un aryle en $C_{6-10}$ qui est éventuellement substitué par 1 à 3 alkyles à chaîne droite ou ramifiée en $C_{1-9}$, halogène, nitro, $CF_3$ ou $SO_3H$ ou des groupes —$OR^{10}$ où $R^{10}$ est un hydrogène ou un alkyle à chaîne droite ou ramifiée en $C_{1-4}$ sous réserve que le nombre total des atomes de carbone du groupe —AY soit de 0 à 20; ou $R^7$ et Y peuvent former ensemble une chaîne polyméthylène en $C_{2-7}$ éventuellement substituée par un ou deux groupes alkyles en $C_{1-4}$ et éventuellement interrompue par des groupes —O—, —S— ou —NR″— où R″ est un alkyle à chaîne droite ou ramifiée en $C_{1-4}$; et $X^1$ est Cl ou Br; comprenant

a) la réaction, éventuellement en présence d'un solvant et d'un agent minéral ou organique fixant les acides, d'un composé répondant à la formule II:

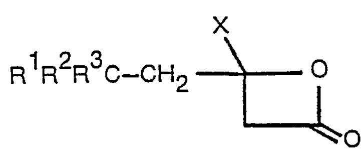

II

dans laquelle $R^2$ et $R^3$ ont leur signification précédente; $R^1$ est un fluor, un chlore ou un brome; et X est un chlore, un brome ou un iode, sous réserve que lorsque X est un chlore aucun de $R^1$, $R^2$, $R^3$, $R^4$, $R^5$ et $R^6$ ne peut être un brome; avec un composé de formule H—Z (III) où Z a sa signification précédente; et

b) lorsque Z est —AY et —AY est —OH, le produit du stade (a) est éventuellement mis à réagir avec un composé (IV) capable de transformer un groupe carboxyle en le chlorure ou bromure d'acide carboxylique correspondant, pour produire ainsi un composé de formule I où Z est —$X^1$.

2. Un procédé selon la revendication 1 dans lequel dans le stade (a), le solvant est soit un solvant inerte dans les conditions de réaction soit un excès d'un des composés réagissants.

3. Un procédé selon la revendication 1 ou 2 dans lequel la température de réaction dans le stade (a) est de −20° à 150°C.

4. Un procédé selon la revendication 1 dans lequel A est O et on fait réagir une lactone de formule II avec un excès d'un alcool de formule III en présence d'un excès de l'agent fixant les acides.

5. Un procédé selon la revendication 1 dans lequel A est $NR^7$ où $R^7$ est comme défini dans la revendication 1 et on agite ensemble des quantités équimoléculaires d'une lactone de formule II et d'une amine de formule III dans un solvant inerte en présence d'un excès d'agent fixant les acides.

6. Un procédé selon la revendication 1 où A est $NR^7$ où $R^7$ est comme défini dans la revendication 1 et on fait réagir une lactone de formule II avec une amine de formule III dans le dioxanne aqueux, l'excès d'amine étant utilisé comme agent fixant les acides.

7. Un procédé selon l'une quelconque des revendications précédentes, où on utilise une lactone de formule II dans laquelle $R^1$, $R^2$, $R^3$ et X sont chacun un chlore ou un brome.

8. Un procédé selon la revendication 7 où $R^1$, $R^2$, $R^3$ et X sont chacun un chlore.

9. Une composition antifongique et antibactérienne comprenant comme ingrédient actif un composé de formule I comme défini dans la revendication 1.

10. Une composition antimicrobienne selon la revendication 9 comprenant une quantité d'un composé de formule I comme défini dans la revendiction 1, qui est efficace contre le développement des bactéries et champignons qui sont pathogènes pour les plantes.

11. Une composition selon la revendication 9 où A est O ou $NR^7$ où $R^7$ est comme défini dans la revendication 1.

12. Une composition selon la revendication 11 où $R^2$ et $R^3$ sont chacun un chlore.

13. Une composition selon la revendication 12 où A est O et Y est H ou un alkyle ayant de 1 à 6 atomes de carbone et éventuellement substitué par 1 à 5 atomes de chlore.

14. Une composition selon la revendication 12 où A est $NR^7$ où $R^7$ est H ou un alkyle ayant de 1 à 6 atomes de carbone.

15. Composés de formule:

$$R^2R^3C{=}CHCOCH_2COZ \qquad IA$$

dans laquelle $R^2$ est un fluor, un chlore ou un brome, $R^3$ est un hydrogène, un fluor, un chlore, un brome ou un groupe de formule $—CR^4R^5R^6$ où $R^4$, $R^5$ et $R^6$ sont un fluor, un chlore ou un brome, Z est —AY où $—X^1$, A est —O—, —S— ou $—NR^7—$ où $R^7$ est un hydrogène, un alkyle à chaîne droite ou ramifiée en $C_{1-10}$ ou un alcényle à chaîne droite ou ramifiée en $C_{3-4}$ et Y est un hydrogène, un alkyle à chaîne droite ou ramifiée en $C_{1-18}$ éventuellement interrompu par 1 à 5 atomes d'oxygène et est éventuellement substitué par 1 à 5 atomes de chlore ou de brome, ou par un groupe de formule $—OR^8$ ou de formule $—CO_2R^9$ où $R^8$ est H, un cycloalkyle en $C_{3-12}$, un alcényle à chaîne droite ou ramifiée en $C_{3-18}$, un aryle en $C_{6-10}$ ou un aralkyle en $C_{7-13}$ et $R^9$ est un hydrogène, un alkyle à chaîne droite ou ramifiée en $C_{1-18}$ ou un alcényle à chaîne droite ou ramifiée en $C_{3-18}$, ou Y est un alcényle à chaîne droite ou ramifiée en $C_{3-18}$, un alcynyle à chaîne droite ou ramifiée en $C_{3-18}$, un cycloalkyle en $C_{3-12}$, un aralkyle en $C_{7-13}$ ou un aryle en $C_{6-10}$ qui est éventuellement substitué par 1 à 3 alkyles à chaîne droite ou ramifiée en $C_{1-9}$, halogène, nitro, $CF_3$, $SO_3H$ ou des groupes $OR^{10}$ où $R^{10}$ est un hydrogène ou un alkyle à chaîne droite ou ramifiée en $C_{1-4}$; sous réserve que le nombre total des atomes de carbone du groupe —AY soit de 0 à 20; ou $R^7$ et Y peuvent former ensemble une chaîne polyméthylène en $C_{2-7}$ éventuellement substituée par un ou deux groupes alkyles en $C_{1-4}$ et éventuellement interrompue par des groupes O, S ou $NR^{11}$ où $R^{11}$ est un alkyle à chaîne droite ou ramifiée en $C_{1-4}$; et $X^1$ est Cl ou Br; si ce n'est que, lorsque $R^2$ et $R^3$ sont chacun un chlore et A est —O—, Y est autre qu'un hydrogène ou un éthyle.

16. Composés selon la revendication 15 où A est —O— ou $—NR^7—$ où $R^7$ est comme défini dans la revendication 1.

17. Composés selon la revendication 16 où $R^2$ et $R^3$ sont chacun un chlore.

18. Composés selon la revendication 17 où A est $NR^7$ où $R^7$ est H ou un alkyle ayant 1 à 6 atomes de carbone.

19. Composés selon la revendication 17 où A est $NR^7$ où $R^7$ est H, $R^2$ et $R^3$ sont Cl et Y est un alkyle en $C_{1-6}$ éventuellement substitué, un alcényle en $C_{2-4}$, un cycloalkyle en $C_{3-6}$, un benzyle ou un phényle.

20. Composés selon la revendication 17 où A est —O— et Y est un méthyle ou un alkyle en $C_{3-6}$.